(19) Europäisches Patentamt
European Patent Office
Office européen des brevets

(11) **EP 4 105 318 B1**

(12) **EUROPEAN PATENT SPECIFICATION**

(45) Date of publication and mention
of the grant of the patent:
**18.09.2024 Bulletin 2024/38**

(21) Application number: **21716480.5**

(22) Date of filing: **12.02.2021**

(51) International Patent Classification (IPC):
**C12N 5/00** (2006.01)

(52) Cooperative Patent Classification (CPC):
**C12N 5/0068;** C12N 2500/60; C12N 2533/54

(86) International application number:
**PCT/ES2021/070101**

(87) International publication number:
**WO 2021/160919 (19.08.2021 Gazette 2021/33)**

(54) **SYSTEM FOR STORING AND TRANSPORTING CELL LINES**

SYSTEM ZUM AUFBEWAHREN UND TRANSPORTIEREN VON ZELLLINIEN

SYSTÈME POUR LE STOCKAGE ET LE TRANSPORT DE LIGNÉES CELLULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.02.2020 PCT/ES2020/070100**

(43) Date of publication of application:
**21.12.2022 Bulletin 2022/51**

(73) Proprietor: **Readycell, S.L.
08005 Barcelona (ES)**

(72) Inventors:
• **SOTO FERNANDEZ, Cristina
08005 BARCELONA (ES)**
• **VAZQUEZ SÁNCHEZ, Maria, Angeles
08005 BARCELONA (ES)**
• **GOMBAU SUÁREZ, Lourdes
08005 BARCELONA (ES)**
• **PALAU LLUCH, Gerard
08005 BARCELONA (ES)**
• **CABRÉ ESTIVILL, Alexandre
08005 BARCELONA (ES)**
• **AUBOUY, Laurent
08005 BARCELONA (ES)**

(74) Representative: **Herrero & Asociados, S.L.
Edificio Aqua - Agustín de Foxá, 4-10
28036 Madrid (ES)**

(56) References cited:
**EP-A1- 1 650 292      US-A1- 2009 042 289**

• **EVANS P J: "Hypothermic preservation of
hepatocytes on gelatin gels", CELL BIOLOGY
INTERNATIONAL, ACADEMIC PRESS, GB, vol.
18, no. 11, 1 January 1994 (1994-01-01), pages 999
- 1008, XP002190794, ISSN: 1065-6995, DOI:
10.1006/CBIR.1994.1023**

**Description**

**FIELD OF THE INVENTION**

The present invention belongs to the field of cell line storage and transport. More particularly, the invention relates to a system for the in vitro storage and transport of cell lines based on the combination of a certain cell transport medium and a gelatin matrix. The system of the invention is especially suitable for transporting HEK-293 and Caco-2 cell lines, since their survival and viability remain substantially unaltered for at least 96 hours of storage and/or transport. The invention is also aimed at a method for the in vitro transport of cell lines, as well a kit for transporting said cell lines.

**BACKGROUND OF THE INVENTION**

Cell cultures, homogeneous or not (co-cultures), can constitute useful models of some genetic, biochemical, metabolic or physiological processes that take place in a living organism. Their ease of use makes it possible to analyze a large number of conditions before carrying out the definitive experiments on animals or clinical assays on humans. In vitro models constitute a tool for validating new therapeutic targets, for selecting seeds in high-performance systems, for defining the action mechanism of new molecules and, in general, for biomedical, biotechnological or cosmetic research.

In general, all cell culture-based models have a limited shelf life. Thus, cultured cells go through various differentiation stages and require continuous manipulation to maintain the properties that make them a suitable model. These limitations in the manipulation and generation of the different in vitro cell models make them difficult to implement for occasional users and, in general, limit the commercialization of the models in their end format.

It must be added to this that, when transporting cell lines, cell survival decreases as the hours pass, which renders the transport of certain cell lines unviable for the commercial marketing thereof in a ready-to-use format. Therefore, there is a need to develop cell line transport systems and/or cell line transport media that make it possible to improve cell survival times and rates to facilitate the storage and transport and, therefore, the commercialization of these ready-to-use cell models.

Some prior art documents describe solutions for improving the transport of cell lines, such as for example EP1650292. However, the transport system and methods described in this prior art document are not optimal for transporting all types of cell lines. For example, the HEK-293 cell line is highly sensitive and has a viability of more than 24 hours, which makes its commercial marketing in ready-to-use format impossible.

Some other solutions require special hypothermic conditions for the transport of cells such as the solutions provided in Evans et al. Cell Biol. Int., 1994, Vol.18 (11): 999 and US2009042289.

Therefore, there is a need to develop new cell line transport systems that make it possible to extend the survival and viability rates of the most sensitive cell lines, such as HEK-293, beyond 48 hours and preferably beyond 72 hours so that this type of cells can be marketed in a ready-to-use format.

The present invention provides an in vitro storage and transport system that enables the survival and viability of different cell lines, particularly the HEK-293 cell line, for periods greater than 72 hours and even up to 96 hours that, in principle, enables the transport of these types of cells in ready-to-use format to any continent. The system of the present invention has proven especially suitable and useful for transporting HEK-293 cells and Caco-2 cell lines.

**OBJECT OF THE INVENTION**

The main object of the invention is a system for the in vitro storage and transport of cell lines comprising:

a) a cell transport medium comprising:

i. 78 % to 88.5 % by volume of Leibovitz L-15 basal medium
ii. 8 % to 12 % by volume of FBS
iii. 2.5 % to 6 % by volume of HEPES
iv. 0.5 % to 2 % by volume of Glutamine
v. 0.5 % to 2 % by volume of a mixture of penicillin and streptomycin

b) a solidified matrix of 10-50 mg of gelatin per ml of transport medium.

Hereinafter, this storage and/or transport system will be referred to as the system of the invention.

Another object of the present invention is a method for the in vitro storage and/or transport of cell lines based on the use of the system of the invention. This method will be referred to as the method of the invention.

A further object refers to a kit comprising the system of the invention and a support for immobilizing the cell line to be transported. This will be referred to as the kit of the invention.

A last object of the invention is represented by the use of the system of the invention for storing and/or transporting cell lines and, preferably, for transporting HEK-293 and Caco-2 cell lines.

## BRIEF DESCRIPTION OF THE FIGURES

**Figure 1:** Comparison about the cell viability of mock HEK-293 after exposure to the transport system of the invention against the usual medium (D6046) for the cultivation of these cells.

**Figure 2:** Comparison about the cell viability of HEK-293 MATE1 after exposure to the transport system of the invention against the usual medium (D6046) for the cultivation of these cells.

**Figure 3:** Comparison on the maintenance of the integrity of the cell monolayer in Caco-2 cells after exposure to the transport system of the invention compared to the current Caco-2 cell transport system (CacoReady).

## DETAILED DESCRIPTION OF THE INVENTION

System for the in vitro storage and transport of cell lines

The main aspect of the invention refers to a system for the in vitro storage and transport of cell lines comprising:

a) a cell transport medium comprising:

i. 78 % to 88.5 % by volume of Leibovitz L-15 basal medium
ii. 8 % to 12 % by volume of FBS
iii. 2.5 % to 6 % by volume of HEPES
iv. 0.5 % to 2 % by volume of Glutamine
v. 0.5 % to 2 % by volume of a mixture of penicillin and streptomycin

b) a solidified matrix of 10-50 mg of gelatin per ml of transport medium.

The storage and transport system of the present invention has surprisingly shown the ability to maintain cell viability beyond 72 hours. In the case of HEK-293 cells, which to date were unable to support shipping times of more than 24 hours, cell survival rates of at least 80 % after 96 hours were observed. In the case of Caco-2 cells, it has been observed that the storage and transport system of the present invention allows the transport of cells at room temperature, maintaining the integrity of the monolayer unchanged for up to at least 11 days.

pH is one of the main parameters that affects cell line viability and, in particular, HEK-293 and Caco-2cells. The authors of the invention have observed that the joint action of HEPES, which is a buffer agent, and the action of the Leibovitz L-15 basal medium provide the system of the invention with the ability to maintain a stable physiological pH so that the cell survival and viability are remarkably improved.

Another essential element of the storage and transport system of the invention is the solidified gelatin matrix. This allows protecting the cells, as a well as preserving their functional properties. Gelatin also guarantees a proper gas exchange of the cells.

Any type of natural or commercial gelatin can be used in the context of the system of the invention, although the gelatin used is preferably type A gelatin.

As mentioned above, the system of the invention allows any cell type to be transported, but is especially suitable for storing and transporting cell lines particularly sensitive to stress situations. In this regard, the system is adapted and especially suitable for transporting HEK-293 cells, as well as Caco-2 cells.

In order to use the system of the invention, firstly the cells must be immobilized on a support for transport. It may be used any type of support that allows cell transport. In a particular embodiment, the support is selected from a Petri dish, a multiwell plate, transwell membrane inserts, biomimetic systems, synthetic supports for 3D cultures, glass plates, slides or any type of support subjected to previous coating so as to facilitate cell adhesion, such as, e.g., a coating with collagen, polylysine or other similar elements.

When the cells reach their proper functional state and the proper confluence state is when they are ready to be coated with the system of the invention. In the context of the present invention, proper functional state is understood to be the state of the viable cells of the culture when they are capable of carrying out their assigned function for the assay for which they are intended. Once they reach said proper functional state and the proper confluence, the system of the invention is added, guaranteeing, thanks to the gelatin, that the cells will be perfectly immobilized and will maintain their functional state until they reach their final destination. Gelatin limits and reduces the mechanical stress to which the cells are subjected during the transport process.

Thanks to the joint action of the transport medium that reduces physiological stress, as well as gelatin that reduces the mechanical stress of the cells, it is produced a synergistic effect that provides the appropriate transport conditions for the cells to reach their destination in its optimal functional state.

The system of the present invention has the advantage that it does not require special transport conditions and, in fact, allows the transport of cell lines at room temperature. This represents an advantage over other systems that require refrigeration or special transport conditions.

Method for storing and/or transporting cell lines

A second aspect of the present invention relates to a method for the in vitro storage and/or transport of cell lines comprising:

A) coating a cell line immobilized on a support with a system comprising:

a) a cell transport medium comprising:

i. Leibovitz L-15 basal medium
ii. FBS
iii. HEPES
iv. Glutamine
v. mixture of penicillin and streptomycin

b) a gelatin matrix.

B) solidifying the gelatin matrix at a temperature between 15 °C and 25 °C
C) storing and/or transporting the cell line at a temperature between 15 °C and 25 °C for a period of up to 96 hours.

In a particular embodiment, the method comprises using:

a) a cell transport medium comprising:

i. 78 % to 88.5 % by volume of Leibovitz L-15 basal medium
ii. 8 % to 12 % by volume of FBS
iii. 2.5 % to 6 % by volume of HEPES
iv. 0.5 % to 2 % by volume of Glutamine
v. 0.5 % to 2 % by volume of a mixture of penicillin and streptomycin

b) a matrix of 10-50 mg of gelatin per ml of transport medium.

The first stage of the method (stage A) is aimed at preparing and accommodating the cell culture to be transferred. To this end, prior to coating the cells with the system of the invention, they are sown at the density determined for each cell type. In accordance with the type of assay for which they are intended, the cell lines will be sown on one type of support or another. The culture must be maintained for as long as necessary until it reaches the suitable functional and confluence, preferably changing the medium every 48-72 hours if necessary.

Although any suitable support for cell transport can be used, in particular the support used is selected from a Petri dish, a multiwell plate, transwell membrane inserts, biomimetic systems, synthetic supports for 3D cultures, glass plates, slides or any type of support previously coated so as to facilitate cell adhesion such as a coating with collagen, polylysine or other similar elements.

Once reached the suitable functional state, the cell line is coated with the system of the invention, comprising the transport medium and the gelatin solution that must be in the liquid state, in accordance with stage A of the method. The gelatin solution is prepared by dissolving gelatin in the transport medium, which acts as a solvent. Thanks to the use of the transport medium as a solvent, it is achieved that the cell line stored and / or transported according to the method of the present invention preserves the functional properties of the culture so that it can be used immediately once it has reached its final destination.

Any type of natural or commercial gelatin can be used in the context of the system of the invention, although the gelatin used is preferably type A gelatin.

In stage B), the gelatin matrix is solidified at a temperature between 15 °C and 25 °C, whereupon the cells are immobilized and stabilized. The gelatin layer represents a physical protection for the cells and allows them to better withstand the mechanical stress resulting from the transport process.

Transport (stage C) is carried out at a temperature between 15 °C and 25 °C and allows transits greater than 48 hours, preferably greater than 72 hours and preferably up to 96 hours.

Once reached their destination and at the moment the immobilized culture is to be used, the plate is incubated with solid gelatin inside a cell incubator until it is completely liquefied, preferably at 37 °C, with 90 % humidity and 5 % $CO_2$ for 1.5 to 4 hours. Then, it is removed by aspiration and the specific culture medium for the cells in question is applied and the cells are incubated at 37 °C with 90 % humidity 15 % $CO_2$ until use.

Although the method of the present invention is applicable to any type of cell line, it is especially suitable for storing and/or transporting cell lines selected from HEK-293 and Caco-2.

Kit for transporting cell lines

In another aspect, the present invention relates to a kit for the in vitro transport and storage of cell lines comprising:

   a. A system for the in vitro storage and/or transport of cell lines according to the invention,
   b. A support for the immobilization the cell line to be transported.

The cell storage and transport system of the kit of the invention comprises both the transport medium and the gelatin matrix in the terms and parameters described above.

Regarding the support, the kit can include any suitable support for cell transport, although in a preferred embodiment the support used is selected from a Petri dish, a multiwell plate, transwell membrane inserts, biomimetic systems, synthetic supports for 3D cultures, glass plates, slides or any type of support previously coated so as to facilitate cell adhesion, such as a coating with collagen, polylysine or other similar elements.

The kit of the present invention is in principle useful for transporting any type of cell line, although it is especially suitable for storing and/or transporting cell lines selected from HEK-293 and Caco-2 cell lines.

Use

A last aspect of the invention is represented by the use of the system of the invention.

Although the system of the invention allows, in principle, the storage and / or transport of any cell line, it has been specially designed and developed for the transport of cell lines that are most sensitive to stress situations.

Preferably, the system of the present invention is useful for the storage and/or transport of HEK-293 and Caco-2 cell lines.

It has been observed that the system of the invention allows maintaining the cell viability of 100 % of the cells for 72 hours and of at least 80 % of the cells after 96 hours or more depending on the cell type. Therefore, the system of the invention allows the shipment and transport of cells and particularly sensitive cells such as HEK-293 and Caco-2 to practically any place on any continent.

The following examples serve to illustrate the invention, but are not intended to limit the scope of the invention in any way.

**EXAMPLES**

**Example 1: Cell viability of mock HEK-293 and HEK-293 MATE1 cells**

A series of assays were carried out on mock HEK-293 and HEK-293 MATE1 cells to test cell viability after 72 hours (3 days) and 96 hours (4 days) using different transport means. As will be seen, in this comparative study the improved survival effect of the transport composition of the invention was demonstrated against the maintenance medium commonly used for this type of cells (initial transport medium).

The following formulations were assayed:

Initial transport medium without Hepes (D6046 + 0 %H):

   - DMEM D6046 Low glucose 85.5% (Sigma);
   - 10% FBS (Biowest);

- L-Glutamine 1% (Lonza);
- Pen / Strep 1% (Lonza); and
- Gelatin Type A 25 mg / ml (Sigma).

Initial transport medium + 5 % Hepes (D6046 + 5 %H):

- DMEM D6046 Low glucose 80.5% (Sigma);
- 10% FBS (Biowest);
- Hepes 5% (Gibco);
- L-Glutamine 1% (Lonza);
- Pen / Strep 1% (Lonza); and
- Gelatin Type A 25mg / ml (Sigma).

New transport medium without Hepes (L15 + 0 %H):

- Leibovitz L-15 medium 85.5% (Sigma);
- 10% FBS (Biowest);
- Glutamine 1% (Lonza);
- Penicillin-Streptomycin 1% (Lonza); and
- Gelatin Type A 25mg / ml (Sigma).

New transport medium + 5 % Hepes (L15 + 5 %H):

- Leibovitz L-15 medium 80.5% (Sigma);
- 10% FBS (Biowest);
- Hepes 5% (Gibco);
- Glutamine 1% (Lonza);
- Penicillin-Streptomycin 1% (Lonza); and
- Gelatin Type A 25 mg / ml (Sigma).

NOTE: % volume / volume

The comparative study was carried out on cells. Both the mock HEK-293 and HEK-293 MATE1 cells were sown at a density of 50,000 cells/well in a Poly-D-Lysine-coated 96-well plate. 24 hours after seeding, having reached total confluence, the medium was removed and 200 $\mu$l/well of transport medium were added. The plate was left to temper inside the flow cabin, sealed with parafilm and then placed in the climatic chamber at 23 °C.

Plates were liquefied after exposure times to the transport medium of 72 and 96 hours. The transport medium was liquefied by introducing the plates at 37 °C in the $CO_2$ incubator for 90 minutes. Once the gelatin was liquefied, the transport medium was removed and HEK-293 maintenance medium was applied (100 $\mu$l/well).

After 24 hours, cell viability was assayed using Alamar Blue (110 $\mu$l/well of 10 % Alamar Blue in a HEK-293 maintenance medium, 2 hours of incubation in $CO_2$ incubator).

The percentage of cell viability was calculated with respect to the net values of RFU (Relative Fluorescence Units) of control cells at time zero prior to the application of the transport medium (TM):

$$\% \ Viability = 100* (Net \ RFU \ post\text{-}TM) \ / \ (Net \ RFU \ pre\text{-}TM)$$

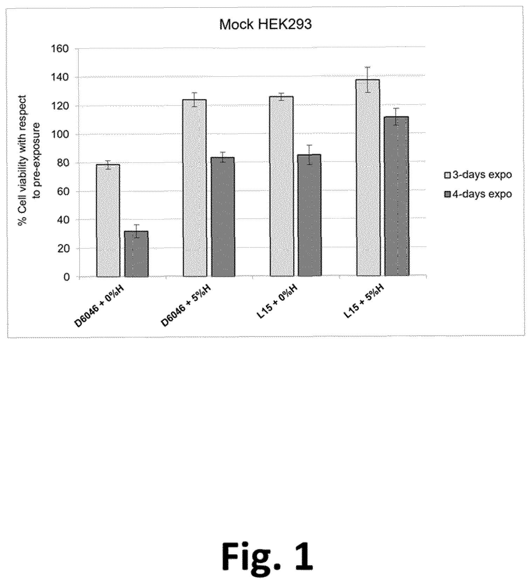

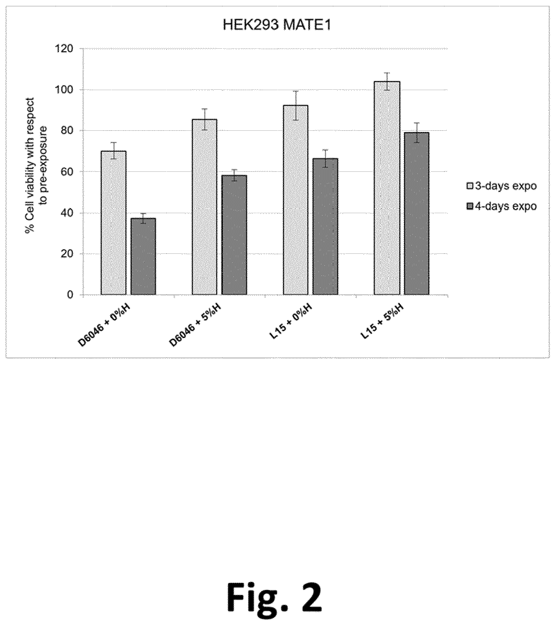

Significant differences between groups were verified using Student's t-test (unpaired). Values of $p \leq 0.05$ were considered statistically significant. Tables 1 and 2 below show the results of the comparative assays for both mock HEK-293 and HEK-293 MATE1 cells. The results can also be observed in Figure 1 (mock HEK-293) and Figure 2 (HEK-293 MATE1).

**Table 1**

| Mock HEK-293 cells | | | |
|---|---|---|---|
| **72 hours of exposure** | | | |
| **Group 1** | **Group 2** | **p-value** | **Statistically Significant** |
| D60+0%H | L15+0%H | 3.5471E-11 | Extremely |
| D60+5%H | L15+0%H | 0.46483386 | No |
| D60+0%H | L15+5%H | 2.3528E-08 | Extremely |
| D60+5%H | L15+5%H | 0.00882694 | Very |
| L15+0%H | L15+5%H | 0.01120801 | Significant |
| **96 hours of exposure** | | | |
| **Group 1** | **Group 2** | **p-value** | **Statistically Significant** |
| D60+0%H | L15+0%H | 2.2275E-08 | Extremely |
| D60+5%H | L15+0%H | 0.66333988 | No |
| D60+0%H | L15+5%H | 1.5208E-10 | Extremely |
| D60+5%H | L15+5%H | 1.6274E-06 | Extremely |
| L15+0%H | L15+5%H | 2.993E-05 | Extremely |

The results with mock HEK-293 cells demonstrate already at 72 hours that the differences between using a Leibovitz L15 basal medium versus DMEM D6046 without the presence of HEPES buffer provides a clear improvement in survival. This difference is minimized when HEPES is added to DMEM D6046 medium but not to Leibovitz L15 basal medium. However, once HEPES is added to the L15 medium, it is observed that the differences with the DMEM D6046 medium are very significant. A significant difference is also observed in the comparison between the L15 medium with or without HEPES. This indicates the importance of this buffer in cell survival. However, taken as a whole, these results suggest that the combination of Leibovitz L-15 basal medium and HEPES buffer is of vital importance to the survival of mock HEK-293 cells after 72 hours.

At 96 hours, the results were similar, although the differences are accentuated, such that the use of Leibovitz L-15 basal medium with 5% HEPES increases cell viability in an extremely significant way compared to the other controls.

**Table 2**

| HEK-293 MATE1 cells | | | |
|---|---|---|---|
| **72 hours of exposure** | | | |
| **Group 1** | **Group 2** | **p-value** | **Statistically Significant** |
| D60+0 %H | L15+0 %H | 5.9771E-05 | Extremely |
| D60+5 %H | L15+0 %H | 0.0913461 | No |
| D60+0 %H | L15+5 %H | 6.2158E-08 | Extremely |
| D60+5 %H | L15+5 %H | 5.1036E-05 | Extremely |
| L15+0 %H | L15+5 %H | 0.00603245 | Very |
| **96 hours of exposure** | | | |
| **Group 1** | **Group 2** | **p-value** | **Statistically Significant** |
| D60+0 %H | L15+0 %H | 4.3599E-08 | Extremely |
| D60+5 %H | L15+0 %H | 0.00270635 | Very |
| D60+0 %H | L15+5 %H | 2.8354E-06 | Extremely |
| D60+5 %H | L15+5 %H | 2.8354E-06 | Extremely |

(continued)

| 96 hours of exposure | | | |
|---|---|---|---|
| Group 1 | Group 2 | p-value | Statistically Significant |
| L15+0 %H | L15+5 %H | 0.00062192 | Very |

The results in HEK-293 MATE1 cells after 72 hours are similar to the results in mock HEK-293 cells, but it is observed that the impact of HEPES on survival is more significant in all comparisons.

At 96 hours, a similar pattern is observed, although what can be inferred is that the relative importance of L-15 basal medium to the survival of HEK-293 MATE1 cells is more significant in the system of the invention than for mock HEK-293 cells. This does not imply that HEPES is unimportant in survival after 96 hours, but again it suggests the synergistic effect between the use of Leibovitz L-15 basal medium together with HEPES buffer in significantly improving cell viability after both 72 hours and 96 hours in HEK-293 MATE1 cells and in mock HEK-293 cells.

In conclusion, the L-15+5 % HEPES composition assayed allows cell transport at room temperature, keeping cell viability substantially unchanged for up to 4 days.

After 4 days of exposure to room temperature, the transport medium based on L15 + 5% HEPES maintains a cell viability of 100% and 80% of HEK-293 mock and MATE1, respectively.

**Example 2: evaluation of cell integrity in Caco-2 cells**

A series of assays were carried out to assess the integrity of the cell layer in Caco2 cells at 4 days (96 hours) and 11 days (264 hours) using different means of transport. The currently available mean of transport allows journeys for a maximum period of 4 days, which is why it was sought to test whether the transport system of the present invention could overcome this time barrier. As will be seen in this comparative study, the improved survival effect of the transport composition of the invention was demonstrated against the current mean. For this, the cell integrity of the monolayer was evaluated after the test period by measuring the transepithelial resistance of the cell monolayer (TEER).

The following formulations were assayed:

Current transport composition (SM CR):

- DMEM Low glucose 85.5% (Sigma);
- 10% FBS (Biowest);
- L-Glutamine 1% (Lonza); and
- Penicillin-Streptomycin 1% (Lonza);
- Gelatin Type A 25mg / ml (Sigma).

Transport composition of the invention (SM L15):

- Medium L-15 (Leibovitz) with L-glutamine 80.5% (Sigma);
- FBS 10% (Biowest);
- Hepes 5% (Gibco);
- L-Glutamine 1% (Lonza);
- Penicillin-Streptomycin 1% (Lonza); and
- Gelatin Type A 25mg / ml (Sigma).

Caco maintenance medium (used for seeding and maintaining the plates):

- DMEM Low Glucose (D6046 Sigma)
- FBS 10% (Biowest)
- L-Glutamine 1% (Lonza)

- Penicillin-Streptomycin 1% (Sigma)

NOTE: % volume / volume

The following equipment was used to carry out the test:

- Biological cabinet
- $CO_2$ incubator
- Climatic chamber
- Epithelial Volt / Ohm (TEER) Meter - EVOM2
- STX100C96 specific electrode for measuring transepithelial electrical resistance in a 96-well transwell plate of the Corning brand

The comparative study was carried out on Caco2 cells, and following the manufacturing procedure stipulated internally for the CacoReady 96-wells product.

Cell seeding was performed at a density of 11,000 cells / well in 4 96-well plates (# 3392, Corning). The cultures were maintained for 13 days, proceeding with changes of medium every 48 hours approximately.

On day 14 after sowing, having reached total confluence, the medium was withdrawn and 75 μL was added in the apical compartment and 235 μL in the basal compartment of transport medium. The plates were allowed to warm inside the flow cabinet, sealed with parafilm and subsequently placed in the climatic chamber at 23°C.

The plates were liquefied after exposure times to the transport medium of 4 days (day 18) and 11 days (day 25). The transport medium was liquefied by placing the plates at 37 ° C in the CO2 incubator for 4 hours. Once the transport medium was liquefied, it was removed and Caco2 maintenance medium (75 μL / apical + 235 μL / basal) was applied.

48 hours after liquefaction, medium changes are made every 2 days until day 25 and 32 of the process, in which the transepithelial resistance of the cell monolayer (TEER) is randomly measured to check the integrity of the cell barrier.

Table 3 summarizes the schedule:

**Table 3**

| SCHEDULE | | | | | | |
|---|---|---|---|---|---|---|
| MONDAY | TUESDAY | WEDNESDAY | THURSDAY | FRIDAY | SATURDAY | SUNDAY |
| D0 SEEDING | D1 | D2 | D3 | D4 | D5 | D6 |
| D7 | D6 | D9 | D10 | D11 | D12 | D13 |
| D14 APPL. SM | D15 | D18 | D17 | D18 LIQUE. SM-1 | D19 | D20 |
| D21 | L22 | C23 | D24 | D25 TEER SM-1 LIQUE. SM-2 | D26 | D27 |
| D28 | D29 | D30 | D31 | D32 TEER SM-2 | | |
| ▪ SM-1: current process - 4 days of exposure ▪ SM-2: tested process - 11 days of exposure | | | | | | |

The transepithelial resistance is calculated relative to the net TEER values by the membrane area of the seed well.

$$TEER = \Omega * cm2$$

- ▪ The membrane area for Corning 96-wells reference # 3392 is equal to 0.143 cm$^2$

- ▪ The CacoReady product specifications establish a correct state of the cell monolayer for a TEER $\geq$ 500 $\Omega$.cm$^2$ measurement

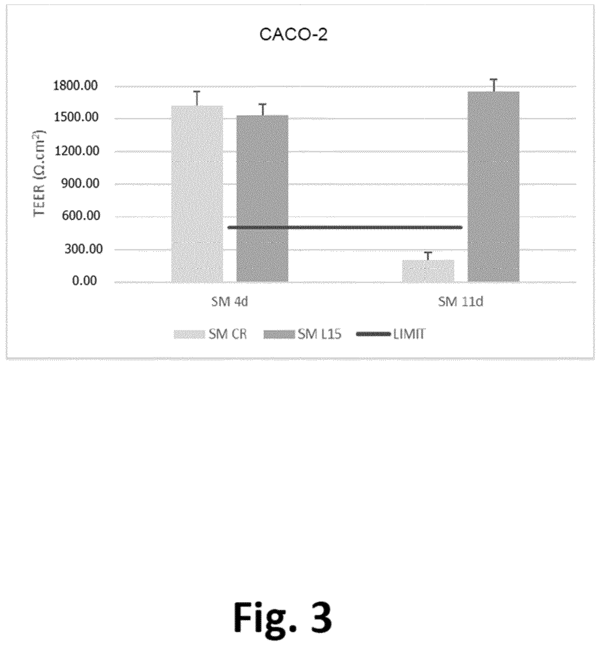

The results obtained on the transepithelial resistance of the cell monolayers for the current transport medium (SM CR) as well as for the transport system of the present invention (SM L15) are detailed in Table 4 for the periods of 4 days and 11 days respectively. These results are also detailed graphically in Figure 3.

**Table 4**

| | | TEER READING ($\Omega$) | | | | | | Average ($\Omega$) | $\Omega$*cm2 | SD |
|---|---|---|---|---|---|---|---|---|---|---|
| EXP 4D | SM CR | 12762 | 10641 | 12240 | 10420 | 12071 | 11500 | 11605.67 | 1624.79 | 129.88 |
| | SM L15 | 10699 | 9710 | 11488 | 11520 | 11401 | 11308 | 11021.00 | 1542.94 | 99.32 |
| EXP 11D | SMCR | 2250 | 1570 | 1740 | 1200 | 925 | 976 | 1443.50 | 202.09 | 71.42 |
| | SM L15 | 12600 | 13200 | 13450 | 12550 | 12240 | 11350 | 12565.00 | 1759.10 | 104.20 |

As can be seen, the current means of transport (SM CR) does not allow the integrity of the monolayer to be maintained for a period of 11 days since the TEER results are outside the established limit specification.

On the contrary, these results demonstrate that the system of the present invention (SM L15) allows the transport of Caco2 cells at room temperature, maintaining the integrity of the monolayer unchanged for at least 11 days, which represents a significant improvement compared to the currently existing composition.

**Claims**

1. A system for the *in vitro* storage and transport of cell lines comprising:

   a) a cell transport medium comprising:

      i. 78 % to 88.5 % by volume of Leibovitz L-15 basal medium
      ii. 8 % to 12 % by volume of FBS
      iii. 2.5 % to 6 % by volume of HEPES
      iv. 0.5 % to 2 % by volume of Glutamine
      v. 0.5 % to 2 % by volume of a mixture of penicillin and streptomycin

   b) a solidified matrix of 10-50 mg of gelatin per ml of transport medium.

2. The system, according to claim 1, wherein the cell lines are selected from HEK-293 and Caco-2 cell lines.

3. The system, according to any of the preceding claims, wherein the solidified matrix is made of type A gelatin.

4. The system, according to any of the preceding claims, wherein the cell line is immobilized on a support for transport thereof.

5. The system, according to claim 4, wherein the support is selected from a Petri dish, multiwell plates, transwell membrane inserts, biomimetic systems, synthetic supports for 3D cultures, glass plates, slides or any type of support previously coated so as to facilitate cell adhesion.

6. The system, according to any of the preceding claims, wherein transport is carried out at room temperature.

7. A method for the in vitro storage and/or transport of cell lines comprising:

   A) coating a cell line immobilized on a support with a system comprising:

      a) a cell transport medium comprising:

         i. Leibovitz L-15 basal medium
         ii. FBS
         iii. HEPES
         iv. Glutamine
         v. mixture of penicillin and streptomycin

b) a gelatin matrix.

B) solidifying the gelatin matrix at a temperature between 15 °C and 25 °C;
C) storing and/or transporting the cell line at a temperature between 15 °C and 25 °C for a period of up to 96 hours.

8. The method, according to claim 7, wherein the system comprises:

a) a cell transport medium comprising:

i. 78 % to 88.5 % by volume of Leibovitz L-15 basal medium
ii. 8 % to 12 % by volume of FBS
iii. 2.5 % to 6 % by volume of HEPES
iv. 0.5 % to 2 % by volume of Glutamine
v. 0.5 % to 2 % by volume of a mixture of penicillin and streptomycin

b) a matrix of 10-50 mg of gelatin per ml of transport medium.

9. The method, according to claim 7 or 8, wherein the support is selected from a Petri dish, multiwell plates, transwell membrane inserts, biomimetic systems, synthetic supports for 3D cultures, glass plates, slides or any type of support previously coated so as to facilitate cell adhesion.

10. The method, according to any of the preceding claims, wherein the solidified matrix is made of type A gelatin.

11. The method, according to any of the preceding claims, wherein the stored and/or transported cell lines are selected from HEK-293 and Caco-2 cell lines.

12. A kit for the in vitro transport and storage of cell lines comprising:

a. A system, according to any of claims 1 to 3 and 5 to 6.
b. A support for immobilizing the cell line.

13. The kit, according to claim 12, wherein the support is selected from a Petri dish, multiwell plates, transwell membrane inserts, biomimetic systems, synthetic supports for 3D cultures, glass plates, slides or any type of support previously coated so as to facilitate cell adhesion.

14. Use of a system, according to claims 1 to 6, for the storage and/or transport of cell lines, preferably HEK-293 and Caco-2 cell lines.

**Patentansprüche**

1. System für die In-vitro-Lagerung und den Transport von Zelllinien, umfassend:

a) ein Zelltransportmedium, das Folgendes umfasst:

i. 78 bis 88,5 Volumenprozent Leibovitz L-15-Basalmedium
ii. 8 bis 12 Volumenprozent FBS
iii. 2,5 bis 6 Volumenprozent HEPES
iv. 0,5 bis 2 Volumenprozent Glutamin
v. 0,5 bis 2 Volumenprozent einer Mischung aus Penicillin und Streptomycin

b) eine verfestigte Matrix aus 10-50 mg Gelatine pro ml des Transportmediums.

2. Das System nach Anspruch 1, wobei die Zelllinien aus HEK-293- und Caco-2-Zelllinien ausgewählt sind.

3. System nach einem der vorhergehenden Ansprüche, wobei die verfestigte Matrix aus Typ-A-Gelatine hergestellt ist.

4. Das System nach einem der vorhergehenden Ansprüche, wobei die Zelllinie auf einem Träger für ihren Transport immobilisiert ist.

5. System nach Anspruch 4, wobei der Träger ausgewählt ist aus einer Petrischale, Multiwell-Platten, Transwell-Membraneinsätzen, biomimetischen Systemen, synthetischen Trägern für 3D-Kulturen, Glasplatten, Objektträgern oder jeder Art von Träger, der zuvor beschichtet wurde, um die Zelladhäsion zu erleichtern.

6. Das System nach einem der vorhergehenden Ansprüche, wobei der Transport bei Raumtemperatur erfolgt.

7. Verfahren zur In-vitro-Lagerung und/oder zum Transport von Zelllinien, umfassend:

A) Beschichten einer auf einem Träger immobilisierten Zelllinie mit einem System, das Folgendes umfasst:

a) ein Zelltransportmedium, das Folgendes umfasst:

i. Leibovitz L-15 Basalmedium
ii. FBS
iii. HEPES
iv. Glutamin
v. Mischung aus Penicillin und Streptomycin

b) eine Gelatinematrix.

B) Verfestigen der Gelatinematrix bei einer Temperatur zwischen 15 °C und 25 °C;
C) Lagern und/oder Transportieren der Zelllinie bei einer Temperatur zwischen 15 °C und 25 °C für einen Zeitraum von bis zu 96 Stunden.

8. Verfahren nach Anspruch 7, wobei das System umfasst:

a) ein Zelltransportmedium, umfassend:

i. 78 % bis 88,5 % des Volumens des Leibovitz L-15 Basalmediums
ii. 8 bis 12 Volumenprozent FBS
iii. 2,5 bis 6 Vol.-% HEPES
iv. 0,5 bis 2 Volumenprozent Glutamin
v. 0,5 bis 2 Volumenprozent einer Mischung aus Penicillin und Streptomycin

b) eine Matrix aus 10-50 mg Gelatine pro ml des Transportmediums.

9. Verfahren nach Anspruch 7 oder 8, wobei der Träger ausgewählt ist aus einer Petrischale, Multiwell-Platten, Transwell-Membraneinsätzen, biomimetischen Systemen, synthetischen Trägern für 3D-Kulturen, Glasplatten, Objektträgern oder jeder Art von Träger, der zuvor beschichtet wurde, um die Zelladhäsion zu erleichtern.

10. Verfahren nach einem der vorhergehenden Ansprüche, wobei die verfestigte Matrix aus Gelatine des Typs A hergestellt ist.

11. Verfahren nach einem der vorhergehenden Ansprüche, wobei die gelagerten und/oder transportierten Zelllinien aus HEK-293- und Caco-2-Zelllinien ausgewählt sind.

12. Ein Kit für den In-vitro-Transport und die Lagerung von Zelllinien, das Folgendes umfasst:

a. Ein System nach einem der Ansprüche 1 bis 3 und 5 bis 6.
b. Ein Träger zur Immobilisierung der Zelllinie.

13. Kit nach Anspruch 12, wobei der Träger ausgewählt ist aus einer Petrischale, Multiwell-Platten, Transwell-Membraneinsätzen, biomimetischen Systemen, synthetischen Trägern für 3D-Kulturen, Glasplatten, Objektträgern oder jeder Art von Träger, der zuvor beschichtet wurde, um die Zelladhäsion zu erleichtern.

14. Verwendung eines Systems nach einem der Ansprüche 1 bis 6 für die Lagerung und/oder den Transport von Zelllinien, vorzugsweise HEK-293- und Caco-2-Zelllinien

**Revendications**

1.  Système pour le stockage et le transport in vitro de lignées cellulaires, comprenant :

    a) un milieu de transport cellulaire comprenant :

        i. 78% à 88,5% en volume de milieu de base Leibovitz L-15
        ii. 8% à 12% en volume de FBS
        iii. 2,5% à 6% en volume de HEPES
        iv. 0,5% à 2% en volume de Glutamine
        v. 0,5% à 2% en volume d'un mélange de pénicilline et de streptomycine

    b) une matrice solidifiée de 10-50 mg de gélatine par ml de milieu de transport.

2.  Système selon la revendication 1, dans lequel les lignées cellulaires sont choisies parmi les lignées cellulaires HEK-293 et Caco-2.

3.  Système selon l'une quelconque des revendications précédentes, dans lequel la matrice solidifiée est constituée de gélatine de type A.

4.  Système selon l'une quelconque des revendications précédentes, dans lequel la lignée cellulaire est immobilisée sur un support pour son transport.

5.  Système selon la revendication 4, dans lequel le support est choisi parmi une boîte de Petri, des plaques multi-puits, des inserts à membrane Transwell, des systèmes biomimétiques, des supports synthétiques pour cultures 3D, des plaques de verre, des lames ou tout type de support préalablement enduit de manière à faciliter l'adhésion cellulaire.

6.  Système selon l'une quelconque des revendications précédentes, où le transport se fait à température ambiante.

7.  Procédé pour le stockage et/ou le transport in vitro de lignées cellulaires, consistant à :

    A) recouvrir une lignée cellulaire immobilisée sur un support avec un système comprenant :

        a) un milieu de transport cellulaire comprenant :

            i. du milieu de base Leibovitz L-15
            ii. du FBS
            iii. de l'HEPES
            iv. de la Glutamine
            v. un mélange de pénicilline et de streptomycine

        b) une matrice de gélatine

    B) solidifier la matrice de gélatine à une température comprise entre 15°C et 25°C ;
    C) stocker et/ou transporter la lignée cellulaire à une température comprise entre 15°C et 25°C pendant une période allant jusqu'à 96 heures.

8.  Procédé selon la revendication 7, dans lequel le système comprend :

    a) un milieu de transport cellulaire comprenant :

        i. 78% à 88,5% en volume de milieu de base Leibovitz L-15
        ii. 8% à 12% en volume de FBS
        iii. 2,5% à 6% en volume de HEPES

iv. 0,5% à 2% en volume de Glutamine

v. 0,5% à 2% en volume d'un mélange de pénicilline et de streptomycine

b) une matrice de 10-50 mg de gélatine par ml de milieu de transport.

**9.** Procédé selon la revendication 7 ou 8, dans lequel le support est choisi parmi une boîte de Petri, des plaques multi-puits, des inserts à membrane Transwell, des systèmes biomimétiques, des supports synthétiques pour cultures 3D, des plaques de verre, des lames ou tout type de support préalablement enduit de manière à faciliter l'adhésion cellulaire.

**10.** Procédé selon l'une quelconque des revendications précédentes, dans lequel la matrice solidifiée est constituée de gélatine de type A.

**11.** Procédé selon l'une quelconque des revendications précédentes, dans lequel les lignées cellulaires stockées et/ou transportées sont choisies parmi les lignées cellulaires HEK-293 et Caco-2.

**12.** Trousse pour le transport et le stockage in vitro de lignées cellulaires, comprenant :

a. Un système selon l'une quelconque des revendications 1 à 3 et 5 à 6.

b. Un support pour immobiliser la lignée cellulaire.

**13.** Trousse selon la revendication 12, dans laquelle le support est choisi parmi une boîte de Petri, des plaques multi-puits, des inserts à membrane Transwell, des systèmes biomimétiques, des supports synthétiques pour cultures 3D, des plaques de verre, des lames ou tout type de support préalablement enduit de manière à faciliter l'adhésion cellulaire.

**14.** Utilisation d'un système selon les revendications 1 à 6, pour le stockage et/ou le transport de lignées cellulaires, de préférence, des lignées cellulaires HEK-293 et Caco-2.

# Fig. 1

# Fig. 2

# Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1650292 A

- US 2009042289 A

**Non-patent literature cited in the description**

- **EVANS et al.** *Cell Biol. Int.,* 1994, vol. 18 (11), 999